# EUROPÄISCHE PATENTANMELDUNG

(11) **EP 0 820 774 A2**
(43) Veröffentlichungstag der Anmeldung: **28.01.1998**
(21) Anmeldenummer: 97108552.7
(22) Anmeldetag: 28.05.1997
(51) Int. Cl.: A61L 27/00, A61K 6/00

(54) **Biohybrides Zahnimplantat**

(30) Priorität: 25.07.1996 DE 19630034
(71) Anmelder: HÜLS AKTIENGESELLSCHAFT, 45764 Marl (DE)
(72) Erfinder: Hill, Frank, Dr., 40822 Mettmann (DE); Hämmerle, Hugo, Dr., 72072 Tübingen (DE); Schindler, Fritz, Dr., 45879 Gelsenkirchen (DE); Scheideler, Lutz, Dr., 53115 Bonn (DE)

(57) **Zusammenfassung**

Biohybrides Zahnimplantat bestehend aus:
- einem Implantatkern aus Titan, einer Titanlegierung oder einem anderen Hartmaterial
- einer Biokompatibilität verleihenden Ummantelung aus Siliziumcarbid oder einem PMMA/Kollagenverbundmaterial
- und einer unmittelbar vor der Implantierung aufzubringenden Außenschicht aus einer gelartigen Matrix (Gel), die Wurzelhautzellen und Wurzelhautzellen stimulierende Faktoren enthält,
wobei die Wurzelhautzellen ohne Vorschaltung eines in vitro-Vermehrungsschrittes in die Matrix eingearbeitet werden.

## Beschreibung

Zähne sind über einen Halteapparat aus Kollagenfasern (Peridontalligament) elastisch im Kieferknochen aufgehängt. Die Elastizität dieses Halteapparates ermöglicht unter anderem eine flexible Anpassung an unterschiedliche Kaubelastungen.

Die heute üblichen Dentalimplantate verwachsen fest mit dem Kieferknochen und entsprechen hinsichtlich Halteapparat nicht dem natürlichen Vorbild. Dentalimplantate gemäß dem Stand der Technik sind Stifte oder Schrauben aus Titan, beschichtetem Titan oder einem keramischen Material, die einen künstlichen Zahnaufbau tragen und in dieser Kombination einen natürlichen Zahn ersetzen.

Das konventionelle Implantat wird vom Zahnarzt in eine Ausbohrung des Kieferknochens gesetzt und dort mechanisch befestigt. Ein Merkmal der marktgängigen Implantate ist ihre spätere knöcherne Integration in den Kieferknochen. Der Knochen wächst an das Implantat heran, umschließt es und hält es so fest Diese starre Integration in den Kieferknochen steht im Gegensatz zu der elastischen Aufhängung des natürlichen Zahnes. Probleme und Komplikationen treten vor allem auf:
- bei der gelegentlich erforderlich werdenden Explantation des Implantates: Es muß dann mit der Knochenfräse gearbeitet werden, was den Kiefer lokal zerstört;
- bei Brückenkonstruktionen zwischen Implantat und natürlichem Zahn: Das starre Implantat und der bewegliche natürliche Zahn haben eine eingeschränkte Kompatibilität als tragende Elemente einer Brücke;
- beim Kauen: Die starre Integration in den Kieferknochen beeinträchtigt die Bißempfindung.

Darüber hinaus unterbleibt in einigen Fällen die Einwachsung des Implantats, und der Kieferknochen bildet sich aufgrund unphysiologischer Belastungen partiell zurück.

Obwohl die Ratte Integration der Implantate in den Kieferknochen Nachteile mit sieh bringt, gibt es Bestrebungen, diesen, als Osseointegration bezeichneten Vorgang durch spezielle Beschichtungen der Implantate noch zu fördern:
- US 4 957 509: Beschichtungen mit Zirkon-, Siliziumnitrid oder Siliziumcarbid
- US 5 178 901: Beschichtung mit einer keramischen Masse aus CaO/TiO₂/P₂O₅/Na₂O
- JP 3 151 978: Keramik aus Al₂O₃/ZrO₂
- WO 9 222 335: Beschichtung mit Calciumphosphat, das durch eine hydrothermale Behandlung in Hydroxylapatit überführt wurde
- EP 0 205 333: Beschichtung mit hydroxylapatithaltigem Polymethacrylat
- DE 38 31 260: Aufsintern von Hydroxylapatit/Tricalciumphosphat mit anschließender Säurebehandlung führt zu einer porösen Hydroxylapatit-Schicht

Im Gegensatz zu diesen Entwicklungen, die auf eine Verbesserung der Osseointegration der Implantate zielen, steht das Verfahren dieser Erfindung, dessen Anwendung zu einer dem natürlichen Vorbild folgenden elastischen Aufhängung des Implantates im Kieferknochen führt. Wesentliche Merkmale zur Erreichung dieses Zieles sind:
- die Besiedlung von Implantatoberflächen mit den Zellen, die die Kollagenfasern des natürlichen Halteapparates bilden (Peridontalfibroblasten und andere Zellen der Wurzelhaut),
- die Schaffung von Bedingungen, die diese Zellen stimulieren,
- der Einsatz von Implantaten, deren Oberflächen mit der Zellbesiedlung und der Stimulierung der Zellen kompatibel sind.

DE 40 40 872 und DE 42 22 296 offenbaren Implantate für den Dentalbereich, wobei menschliche Peridontalfibroblasten von extrahierten Zahnwurzeln entnommen, in vitro über mehrere Wochen vermehrt und dann kurz vor dem Einsetzen des Implantates auf das Implantat übertragen werden.

Aufgabe der vorliegenden Erfindung war es, ein natürlich einwachsendes Zahnimplantat bereitzustellen, das ohne die in vitro-Vermehrung der Peridontalfibroblasten auskommt.

Gegenstand der vorliegenden Erfindung ist ein biokompatibles Zahnimplantat bestehend aus:
- einem Implantatkern aus Titan, einer Titanlegierung oder einem anderen Hartmaterial
- einer Biokompatibilität verleihenden Ummantelung aus Siliziumcarbid oder einem PMMA/Kollagenverbundmaterial
- und einer unmittelbar vor der Implantierung aufzubringenden Außenschicht aus einer gelartigen Matrix (Gel), die Wurzelhautzellen und Wurzelhautzellen stimulierende Faktoren enthält,
wobei die Wurzelhautzellen ohne Vorschaltung eines in vitro-Vermehrungsschrittes in die Matrix eingearbeitet werden.

Im Rahmen der Untersuchungen zur Lösung dieser Aufgabe wurde ein Zahnimplantat gefunden, bei dem durch Einsatz von speziell behandelten Implantatoberflächen und Stimulantien, vor allem von Mitosestimulantien, die Peridontalfibroblasten sich wesentlich schneller vermehren als im unbehandelten Zustand. Die Stimulierung der Zellaktivität ist überraschenderweise derart hoch, daß bei Einsatz der Stimulantien auf die von DE 40 40 872 und DE 42 22 246 beschriebene in vitro-Vermehrung der Peridontalfibroblasten als Voraussetzung für die Besiedlung der Implantate mit diesen Zellen unterbleiben kann. Somit kann germäß dieser Erfindung in einer Behandlungssitzung:
- der kranke oder abgestorbene Zahn extrahiert werden,
- das Implantat mit durch Probeexcision erhaltenen oder von der Wurzelhaut des extrahierten Zahnes entnommenen Peridontalfibroblasten besiedelt werden,
- das so behandelte Implantat implantiert werden.

Erfindungswesentlich ist die Beschichtung des Zahnimplantats mit einem Wurzelhautzellen und Stimulantien enthaltenden Gel. Dieses Gel laßt sich nach folgender Methode herstellen:
- Proteolytische Behandlung der Zahnwurzelhaut, die durch Probeexcision oder durch Abschaben von einer extrahierten Zahnwurzel erhalten wurde, mit Kollagenase und/oder Trypsin und/oder anderen Enzymen. Die proteolytische Behandlung bewirkt die Auflösung des Gewerbeverbandes. Alternativ zur proteolytischen Behandlung bzw. in Kombination mit ihr kann die Zahnwurzelhaut auch mechanisch zerkleinert werden.
- Konzentrieren und Waschen der erhaltenen Zellsuspension bzw. Gewebesuspension durch Zentrifugation
- Zugabe von Blutplasma oder Serum bzw. von Zellwachstumshormonen wie EGF und/oder BMP und/oder weiteren Faktoren mit mitogener Aktivität auf Wurzelhautzellen
- Zugabe von einem oder mehreren Antibiotika
- Zugabe einer gelbildenden Substanz in Form einer Lösung oder als lösliche Substanz
- Zugabe einer anorganischen Substanz, die die Gelbildung auslöst und/oder stabilisiert oder andersseitig eine positive Wirkung auf die Ausbildung des natürlichen Zahnhalteapparates hat.

Das Gel enthält also neben den Wurzelhautzellen die folgenden Komponenten:
A) eine Gel-bildende Substanz,
B) ein oder mehrere antibiotisch wirksame Substanzen,
C) gereinigte oder in komplexer Mischung vorliegende Faktoren, die die Zellaktivität stimulieren,
D) anorganische Substanzen.

Als gelbildende Substanz werden z. B. Kollagen, Fibrinogen, Aprotinin und/oder Natriumalginat verwendet.

Die Komponente B enthält Wirkstoffe aus der Klasse der Penicilline, Cephalosporine, Streptomycine, Ciprofloxazine, Tetracyline und/oder Erythromycine in Konzentrationen von 10 - 1 000 µg pro ml.

Als stimulierende Faktoren werden reine Proteine aus der Gruppe der epidermalen Zellwachstumsfaktoren, der Knochen-morphogenetischen Proteine und/oder komplexe Extrakte aus juvenilen Zahnknospen von Nutztieren oder fötalem Nutztierserum eingesetzt.

Die anorganischen Substanzen sind vorzugsweise Calciumphosphat, insbesondere Hydroxylapatit.

Das Gel ist mit einer Schichtdicke von 0,1 - 2 mm auf der Implantatoberfläche aufgebracht.

Mit dem so hergestellten Wurzelhautzellen und Stimulantien enthaltenen Gel werden Implantate unmittelbar vor ihrer Implantation beschichtet. Die hierfür besonders geeigneten Implantate haben eine zur Besiedlung mit Wurzelhautzellen und zur Verankerung der von diesen Zellen zu bildenden Kollagenfasern geeignete Oberfläche. Beispiele hierfür sind Implantate, die aus dem PMMA/Kollagenverbundmaterial gemaß der Deutschen Patentanmeldung "Biokompatibles Verbundmaterial und Verfahren zu seiner Herstellung" (Anmeldenummer: 195 29 036.4) hergestellt werden oder mit Siliziumcarbid beschichtete Implantate. Diese werden bevorzugt so hergestellt, daß zur Ausbildung der Implantatoberfläche aus Siliziumcarbid eine künstliche Zahnwurzel, die aus Titan oder dessen Legierung besteht, mit einem Silizium-haltigen Teig überzogen und der Teig zunächst bei Temperaturen < 1 000 °C zersetzt und anschließend bei Temperaturen > 1 400 °C zu SiC umgesetzt wird.

### Detaillierte Beschreibung der Erfindung

A. Herstellung mit Siliziumcarbid beschichteter Implantate
   Ein Mehlteig mit 30 % Mehl und 70 % Siliziumpulver wird mit Wasser zu einem pastösen Brei angerührt und aufeinen Titanstift, der zur Verwendung als künstliche Zahnwurzel vorgesehen ist, aufgetragen. Nach der Lehre der EP 0 555 760 wird diese Masse 1 - 2 h bei einer Temperatur von 200 - 800 °C zersetzt und anschließend bei einer Reaktionstemperatur von > 1 400 °C 1 - 2 h zu SiC umgesetzt. Es entsteht ein Metallstift mit einer rauhen vergüteten Oberfläche aus Siliciumcarbid, die in der Lage ist, neugebildete Kollagenproteine zu binden und so die Ausbildung der Fibrillenstruktur des Peridontiums unterstützt.
B. Bereitung des Gels, das vitale Wurzelhautzellen und Stimulantien enthält
   Die Wurzelhaut eines frisch extrahierten Zahnes wird mit dem Skalpell abgeschabt und in einigen ml Eagles Minimal Essential Medium (MEM) mit einem Zusatz von 30 % fötalem Kälberserum aufgenommen. Nach Zugabe von Trypsin und/oder Kollagenase wird dieser Ansatz ca. 1 h unter Schütteln bei 37 °C inkubiert, wobei die Suspension aus Stücken von Wurzelhautgewebe in eine Suspension vereinzelter Zellen übergeht. Zur Entfernung überschüssigem Enzyms wird die Zellsuspension durch ein Netz mit 50 µm weiten Maschen filtriert und anschließend 5 Minuten bei 150 Upm zentrifugiert. Das zellhaltige Sediment liefert nach zweimaligem Waschen in einigen ml des angegebenen Mediums (MEM + 30 % fötales Kälberserum) einige ml Medium mit vereinzelten und weitgehend gereinigten Wurzelhautzellen.
   Zusatz von Antibiotika
   Der Zellsuspension werden Antibiotika in einer Menge zugesetzt, die auch unter Berücksichtigung der Diffusionsverluste nach Implantation der mit dem Gel ummantelten künstlichen Zahnwurzel eine Sepsis über mehrere Tage verhindert. Besonders geeignete Antibiotika sind Penicillin und Streptomycin sowie weitere, vornehmlich lipophile Antibiotika.
   Zusatz von Wachstumsfaktoren
   Der Zellsuspension werden weiterhin ausreichende Mengen des handelsüblichen "epidermalen Wachstumsfaktor" oder des nach der Methode von Nakae et al. zu isolierenden Wachstumsfaktors zugesetzt (isolation and partial characterization of mitogenic factors from cementum: Biochemie 30, 7047-7052 (1991). Andere gereinigte Wachstumsfaktoren, die zugesetzt werden können, sind der morphogenetische Knochenwachstumsfaktor von Nakashima (Arch. Oral. Biol. 35, 493-497 (1990) oder ein Extrakt aus juvenilen Zahnknospen von Nutztieren (EP 0 263 088). Es kann vorteilhaft sein, zusammen mit den Wachstumsfaktoren Dextran oder Calciumphosphat zuzusetzen, um eine Retardwirkung zu erzielen (EP 0 530 458, JP 6 3105765).
   Ausbildung des Gels
   Die die Wirkstoffe enthaltende Zellsuspension kann auf mehreren Wegen in ein Gel überführt werden:
   - Zusatz von Fibrinogen, Thrombin und Aprotinin
   - Zusatz von Kollagen nach Parson-Wingerter und Saltzman (Biotechnol. Prog. 9, (1993), S. 600 - 607),
   - Zusatz einer Mischung aus Kollagen und Calciumphosphat
   - Zusatz von Natriumalginat und anschließende Auslösung der Gelbildung durch Zugabe von CaCl₂.
C. Ummantelung des Implantates mit dem Gel
   Das Implantat wird z. B. mit Hilfe eines Spatels mit einer 0,1 - 2 mm dicken Schicht des Gels ummantelt.
D. Vereinfachtes Verfahren zur Präparation eines erfindungsgemäßen Implantates
   Wurzelhautstücke werden durch Probeexcision bzw. von der Wurzelhaut eines extrahierten Zahnes gewonnen, mechanisch zerkleinert und in Pufferlösung gewaschen. Die überstehende Waschlösung wird abgezogen und die verbleibenden Gewerbestückchen werden in Kulturmedium - das Wachstumsfaktoren und/oder fötales Kälberserum und Antibiotika enthält - suspendiert und anschließend mit einer gelbildenden Mischung versetzt.
   Als günstig hat sich die wie folgt herzustellende gelbildende Mischung erwiesen: Eine auf 4 °C gekühlte, essigsaure (0,1 n) Kollagenlösung (1 bis 20 mg Kollagen pro ml) wird mit alkalisch eingestelltem Kulturmedium auf einen pH-Wert von 7,4 gebracht. Zu dieser Lösung gibt man die Gewebesuspension im Verhältnis von vorzugsweise 1 Teil Gewebesuspension zu 9 Teilen gelbildende Lösung und vermischt gründlich. In diese Suspension wird dann das zu beschichtende Implantat getaucht. Nach Erwärmung auf Raumtemperatur geliert die Suspension in wenigen Minuten und überzieht das eingetauchte Implantat mit einer zellhaltigen Gelschicht
E. Implantation
   Das mit dem Gel ummantelte Implantat wird in die leere Alveole oder in eine in den Kiefer gefräste Bohrung gesetzt und anschließend werden die Lappen des Zahnfleisches mit einer chirurgischen Naht verschlossen. In der natürlichen Umgebung setzt eine Proliferation der Zellen und die Bildung der Kollagenfasern des Halteapparates ein. Es kann sich als nützlich oder notwendig erweisen, bereits nach 12 - 14 Tagen die Zahnkrone auf das Implantat zu montieren und mit einer provisorischen Schiene oder Drahtschlinge zu stabilisieren. Bevorzugt werden jedoch Einheilzeiten von 4 bis 8 Wochen, bevor die Krone montiert wird.
   Alternativ zu dem oben beschriebenen Vorgehen (lastfreie, schleimhautgedeckte Einheilung) kann die künstliche Zahnwurzel auch bereits vor der Implantation mit einem durch die Schleimhaut in die Mundhöhle ragenden Aufbau versehen werden. Über diesen Aufbau wird das Implantat an die Nachbarzähne in einer Weise fixiert, die einen definierten, geringen Bewegungsspielraum zuläßt (Einheilung unter definierter Belastung).

### Vorteil dieser Erfindung gegenüber DE 40 40 872 und DE 42 22 296

Gegenüber DE 40 40 872 und DE 42 22 296 entfällt die mehrwöchige in vitro-Vermehrungsphase der Wurzelhautzellen und die Extraktion eines nicht mehr erhaltungswürdigen Zahnes kann zusammen mit dem Einsatz eines Implantats in einer Behandlungssitzung vorgenommen werden.

## Patentansprüche

1. Biohybrides Zahnimplantat bestehend aus:
- einem Implantatkern aus Titan, einer Titanlegierung oder einem anderen Hartmaterial
- einer Biokompatibilität verleihenden Ummantelung aus Siliziumcarbid oder einem PMMA/Kollagenverbundmaterial
- und einer unmittelbar vor der Implantierung aufzubringenden Außenschicht aus einer gelartigen Matrix (Gel), die Wurzelhautzellen und Wurzelhautzellen stimulierende Faktoren enthält,
wobei die Wurzelhautzellen ohne Vorschaltung eines in vitro-Vermehrungsschrittes in die Matrix eingearbeitet werden.

2. Biohybrides Zahnimplantat,
dadurch gekennzeichnet,
daß das Gel neben vitalen Zellen eine oder mehrere der folgenden Komponenten enthält:
A) eine Gel-bildende Substanz,
B) ein oder mehrere antibiotisch wirksame Substanzen,
C) gereinigte oder in komplexer Mischung vorliegende Faktoren, die die Zellaktivität stimulieren,
D) anorganische Substanzen

3. Biohybrides Zahnimplantat,
dadurch gekennzeichnet,
daß als Gel-bildende Substanzen Kollagen, Fibrinogen, Aprotinin und/oder Natriumalginat verwendet werden.

4. Biohybrides Zahnimplantat,
dadurch gekennzeichnet,
daß das Gel als antibiotische Substanzen Wirkstoffe aus der Klasse der Penicilline, Cephalosporine, Streptomycine, Ciprofloxazine, Tetracyline und/oder Erythromycine in Konzentrationen von 10 - 1 000 µg pro ml, bzw. 10 - 1 000 I. U./ml enthält.

5. Biohybrides Zahnimplantat,
dadurch gekennzeichnet,
daß als stimulierende Faktoren ausgewählt aus Proteinen, Teilen von Proteinen oder Proteingennischen aus der Gruppe der epidermalen Zellwachstumsfaktoren, der Knochen-morphogenetischen Proteinen und/oder komplexen Extrakten aus juvenilen Zahnknospen von Nutztieren oder fötalem Nutztiersetum eingesetzt werden.

6. Biohybrides Zahnimplantat,
dadurch gekennzeichnet,
daß das GeI anorganische Salze, ausgewählt aus Calciumphosphat und/oder Nydroxylapatit, enthält.

7. Biohybrides Zahnimplantat,
dadurch gekennzeichnet,
daß die Zellen aus Wurzelhautstücken, die von einer extrahierten Zahnwurzel abgeschabt wurden, durch Behandlung mit proteolytischen Enzymen vereinzelt werden und nach dieser Behandlung in Konzentrationen von 10² bis 10⁶ vitalen Zellen pro ml in der Zellsuspension vorliegen.

8. Biohybrides Zahnimplantat,
dadurch gekennzeichnet,
daß die Wurzelhautstücke, die von einer extrahierten Zahnwurzel abgeschabt wurden oder durch Probeexision erhalten wurden, mechanisch zerkleinert werden und nach dieser Zerkleinerung in einer Dichte von 10¹ bis 10³ Stückchen pro ml Suspension vorliegen.

9. Biohybrides Zahnimplantat,
dadurch gekennzeichnet,
daß das Gel mit einer Schichtdicke von 0,1 bis 2 mm auf der Implantatoberfläche vorliegt.

10. Biohybrides Zahnimplantat,
dadurch gekennzeichnet,
daß das mit dem Gel zu beschichtende Implantat eine Oberfläche aus Siliziumcarbid hat.

11. Biohybrides Zahnimplantat,
dadurch gekennzeichnet,
daß das mit dem Gel zu beschichtende Implantat zumindest an seiner Oberfläche aus einem PMMA/Kollagenverbundmaterial besteht.

12. Biohybrides Zahnimplantat,
dadurch gekennzeichnet,
daß zur Ausbildung der Implantatoberfläche aus Siliziumcarbid eine Künstliche Zahnwurzel, die aus Titan oder dessen Legierung besteht, mit einem Siliziumhaltigen Teig überzogen und der Teig zunächst bei Temperaturen < 1 000 °C zersetzt und anschließend bei Temperaturen > 1 400 °C zu SiC umgesetzt wurde.
